(19) Europäisches Patentamt
European Patent Office
Office européen des brevets

(11) Publication number: **0 222 523**
**A2**

(12) # EUROPEAN PATENT APPLICATION

(21) Application number: 86308097.4

(22) Date of filing: 20.10.86

(51) Int. Cl.⁴: C 07 K 5/06
A 61 K 37/64

(30) Priority: 01.11.85 PC T/US85/02177

(43) Date of publication of application:
20.05.87 Bulletin 87/21

(84) Designated Contracting States:
AT BE CH DE ES FR GB GR IT LI LU NL SE

(71) Applicant: PFIZER INC.
235 East 42nd Street
New York, N.Y. 10017(US)

(72) Inventor: Hoover, Dennis Jay
5, Fargo Drive
Ledyard Connecticut(US)

(74) Representative: Moore, James William, Dr.
Pfizer Limited Ramsgate Road
Sandwich Kent CT13 9NJ(GB)

(54) **Difluorocyclostatine containing polypeptides.**

(57) Polypeptides and derivatives thereof containing 2,2-difluorocyclostatine are useful for inhibiting the angiotensinogen-cleaving action of the enzyme renin.

EP 0 222 523 A2

Croydon Printing Company Ltd

PATENT
DPC 7012

# DIFLUOROCYCLOSTATINE CONTAINING POLYPEPTIDES

The proteolytic enzyme renin, which has a molecular weight of about 40,000, is produced in and secreted into the blood by the kidney. It is known to be active *in vivo* in cleaving the naturally-occurring plasma glycoprotein angiotensinogen, in the case of human angiotensinogen at the bond between the leucine (10th) and valine (11th) amino acid residues at the N-terminal end of the angiotensinogen:

| Asp- | Arg- | Val- | Tyr- | Ile- | His- | Pro- | Phe- | His- | Leu- | Val- |
|---|---|---|---|---|---|---|---|---|---|---|
| 1 | 2 | 3 | 4 | 5 | 6 | 7 | 8 | 9 | 10 | 11 |

| Ile- | His- | Ser- | Glu- |
|---|---|---|---|
| 12 | 13 | 14 | 15 |

The circulating N-terminal decapeptide (angiotensin I) formed by the above cleaving action of renin is subsequently broken down by the body to an octapeptide known as angiotensin II. Angiotensin II is known to be a potent pressor substance, i.e., a substance that is capable of inducing a significant increase in blood pressure, and is believed to act by causing the constriction of blood vessels and the release of the sodium-retaining hormone aldosterone from the adrenal gland. Thus, the renin-angiotensinogen system has been implicated as a causative factor in certain forms of hypertension and congestive heart failure.

One means of alleviating the adverse effects of the functioning of the renin-angiotensinogen system is the administration of a substance capable of inhibiting the angiotensinogen-cleaving action of renin. A number of such substances are known, including antirenin antibodies, pepstatin and naturally-occurring phospholipid compounds. European Patent Application No. 45,665 (published February 2, 1982) discloses a series of

renin-inhibiting polypeptide derivatives of the formula

X-Y-Pro-Phe-His-A-B-Z-W

in which X may be hydrogen or an amino-protecting group, Y may be absent, B is a lipophilic amino acid residue, Z is an aromatic amino acid residue, W may be hydroxyl and A may be, inter alia,

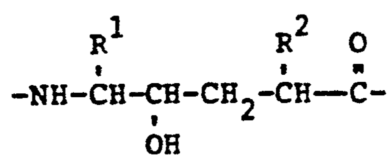

with each of $R^1$ and $R^2$ being a lipophilic or aromatic side chain. According to the definitions set forth in this published patent application, it is not contemplated that either A or Z could be statine or that B could be lysine.

European Patent Application No. 77,028A (published April 20, 1983) discloses a series of renin-inhibiting polypeptide compounds having a non-terminal statine or statine derivative residue. Included within this series are compounds having a phenylalanine-histidine-statine sequence.

European Patent Application 132,304A also discloses the use of statine containing polypeptides as renin-inhibiting antihypertensive agents, and European Patent Application 114,993A discloses polypeptides containing cyclostatine, useful as renin-inhibiting antihypertensive agents.

Certain polypeptides containing fluoroketones related to statine are reported to be inhibitors of pepsin (Gelb, et al., Biochemistry, 24, 1814 (1985).

It has now been found that certain polypeptides containing 5-cyclohexyl-(S)-4-amino-(S) or (R)-3-hydroxy-2,2-difluoropentanoic acid and 5-cyclohexyl-4(R,S)-amino-3-oxo-2,2-difluoropentanoic acid are useful as renin-inhibiting agents and have application in the treatment of hypertension and congestive heart failure.

This series of novel compounds consist of polypeptides and polypeptide derivatives of the formula

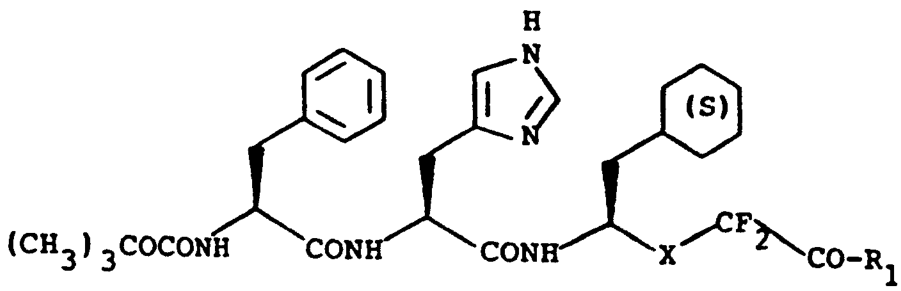

and a pharmaceutically acceptable acid addition salt thereof, wherein X is

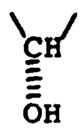 or 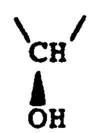

and $R_1$ is alkoxy having one to three carbon atoms or alkylamino having one to three carbon atoms.

Preferred are compounds where $R_1$ is said alkylamino. Especially preferred are compounds where X is 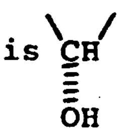 and 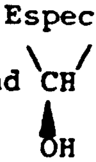 and $R_1$ is methylamino.

Also preferred are compounds where $R_1$ is said alkoxy. Especially preferred is the compound where X is 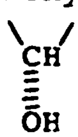 and $R_1$ is ethoxy.

This series of compounds also include the ketones of the formula

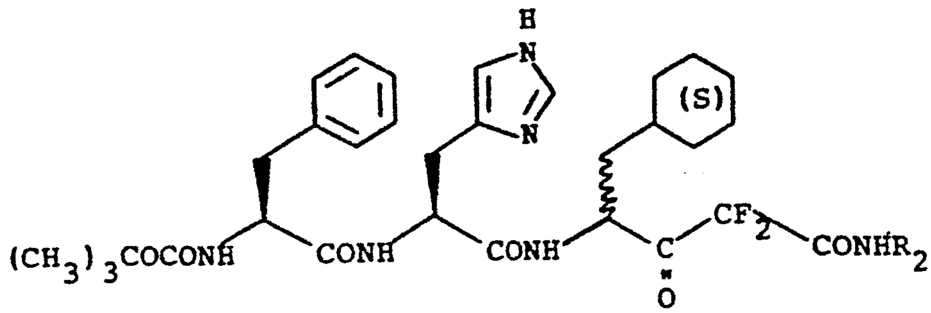

and a pharmaceutically acceptable acid addition salt thereof where $R_2$ is alkyl having one to three carbon atoms.

Preferred is the compound where $R_2$ is methyl.

As previously indicated, the present invention embraces pharmaceutically acceptable salts of the biologically active compounds. Such salts are those which are non-toxic at the dosages administered. Since compounds of the invention may contain both basic and acidic groups, both acid addition and alkali addition salts are possible. Pharmaceutically acceptable acid addition salts include e.g., the hydrochloride, hydrobromide, hydroiodide, sulfate, bisulfate, phosphate, acid phosphate, acetate, lactate, maleate, mesylate, fumarate, citrate, acid citrate, tartrate, bitartrate, succinate, gluconate and saccharate salts. Pharmaceutically acceptable alkali addition salts include e.g., the sodium, potassium, calcium and magnesium salts. Conventional methods of forming acid addition and alkali addition salts may be employed.

In the interest of brevity, the commonly accepted abbreviated name of the individual aminoacids have been employed where possible. For example, the amino acid phenylalanine is abbreviated as Phe and histidine as His. The aminoprotecting group *t*-butoxycarbonyl is abbreviated as Boc and N-*t*-butoxycarbonyl on the imidazole of histidine as imBoc.

The modified cyclostatine containing fluorine in the structure is of the formula

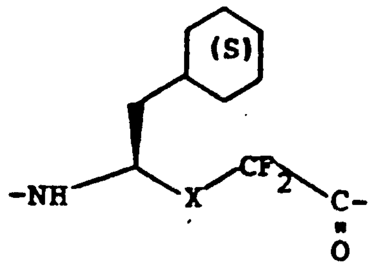

where X is as previously described. These structures are designated 2,2-difluorocyclohexylSta where X is 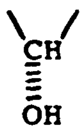 and 2,2-difluoro-epi-cyclohexylSta where X is 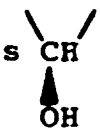. The corresponding ketones are designated 2,2-difluoro-3-oxocyclohexylSta.

All the natural amino acid contained in the structures of the instantly claimed compounds are of the L configuration, the naturally occurring configuration, unless otherwise noted.

Also considered within the scope of the present invention and prepared in a manner analogous to the instantly claimed compounds are therapeutic agents of the formula

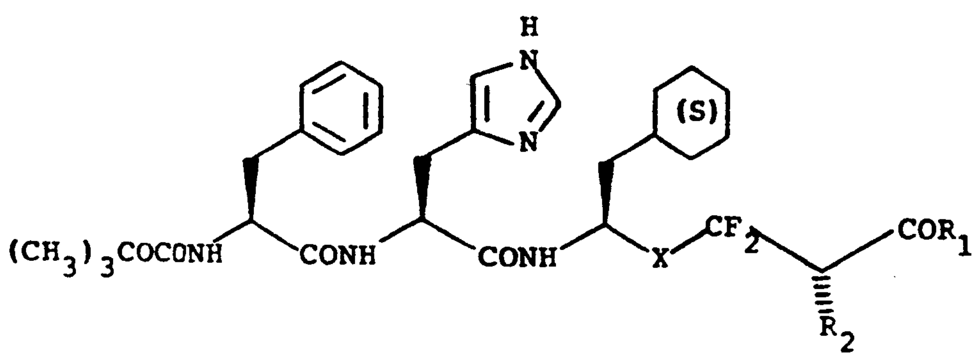

where $R_1$ is as herein defined, $R_2$ is isobutyl or benzyl and Y is 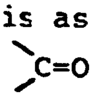 or 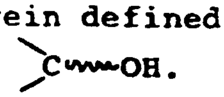.

The compounds of this invention exhibit antihypertensive activity *in vivo* in mammals, including humans. At least a substantial portion of this activity results from their ability to inhibit the cleavage of angiotensinogen by renin. Although we do not wish to be limited by the following theory of mechanism, it is likely that the mechanism of the renin-inhibiting activity of the compounds of the invention is their selective binding (as compared to angiotensinogen) to renin. The compounds of the invention exhibit an enzyme-inhibiting activity that is selective for renin as against other beneficial enzymes such as cathepsin D. Because of their low molecular weights they exhibit favorable solubility characteristics in aqueous media, thus making oral administration feasible, and can be synthesized at a commercially realistic cost. The compounds of the present invention are also useful against congestive heart failure.

The compounds of the invention may be prepared by methods familiar to those skilled in the art. The basic sub-unit of the preferred chemical synthesis is the acylation of the unprotected alpha-amino group of an amino acid residue with an amino acid having an activated (for acylation purposes) carboxylic function and a suitable protecting group bonded to its own alpha-nitrogen to form a peptide bond between the two amino acid residues, followed by the removal of said protecting group. This synthesis sub-unit of coupling-deblocking is performed repeatedly to build up the polypeptide, starting from the C-terminal end of the molecular structure and working to the N-terminal end as described herein. The amino acids utilized to synthesize the compounds of the present invention are commercially available (as free acids, salts or esters, etc.) in both alpha-amino protected and alpha-amino unprotected forms.

Synthesis of the intermediate forming the skeleton of 2,2-difluorocyclohexylSta starts with the coupling of N-t-butoxycarbonyl-L-phenylalaninal with ethyl bromodifluoroacetate to give a mixture of the 3-S and R epimers of the structure

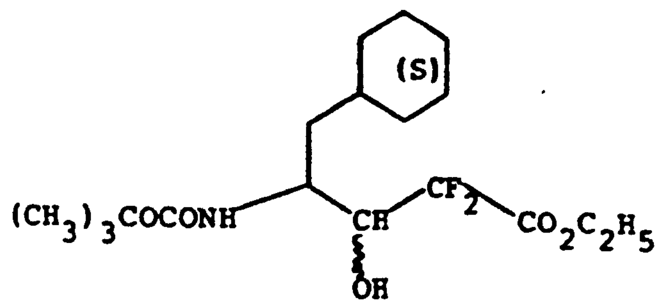

Ammonolysis of the resulting ester with an alkylamine leads to the corresponding N-alkylamide.

The amides or esters, when subjected to hydrogen chloride in dioxane, lose the t-butoxycarbonyl protecting group from the amino moiety. Acylation of the resulting amino esters or amides with BocPheHis(imBoc) using 1-hydroxybenzotriazole and dicyclohexylcarbodiimide gives the penultimate compound in the sequence. Removal of the blocking group on imidazole with acetic acid-water gives the final product.

The ketone containing polypeptides of the present invention are prepared by oxidation of the corresponding (R) or (S) hydroxy polypeptides using dimethylsulfoxide and trichloroacidic anhydride.

The activity of the compounds of the present invention as inhibitors of the angiotensinogen-cleaving activity of renin may be determined by studying (1) their ability to inhibit the angiotensinogen-cleaving activity of renin *in vitro* and (2) their ability to antagonize the exogenous renin-induced pressor response *in vivo*.

The compounds of the present invention can be administered as antihypertensive agents by either the oral or parental routes of administration, with the

former being preferred for reasons of patient convenience and comfort. In general, these antihypertensive compounds are normally administered orally in dosages ranging from about 0.5 mg to about 50 mg per kg of body weight per day and 0.1 mg to about 5 mg per kg of body weight per day when given parenterally; variations will necessarily occur depending upon the condition of the subject being treated and the particular compound being administered. Typically, treatment is commenced at a low daily dosage and increased by the physician only if necessary. It is to be noted that these compounds may be administered in combination with pharmaceutically acceptable carriers by either of the routes previously indicated, and that such administration can be carried out in both single and multiple dosages.

The novel compounds of the invention can be orally administered in a wide variety of different dosage forms, i.e., they may be formulated with various pharmaceutically acceptable inert carriers in the form of tablets, capsules, lozenges, troches, hard candies, powders, sprays, aqueous suspensions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. In general, the compounds of this invention are present in such oral dosage forms at concentration levels ranging from about 0.5% to about 90% by weight of the total composition, in amounts which are sufficient to provide the desired unit dosages.

For purposes of oral administration, tablets containing various excipients such as sodium citrate, calcium carbonate and calcium phosphate may be employed along with various disintegrants such as starch and preferably potato or tapioca starch, alginic acid and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred materials in this connection would also include lactose or milk sugar as well as high molecular weight polyethylene glycols. When aqueous suspensions and/or elixirs are desired of oral administration, the essential active ingredient therein may be combined with various sweetening or flavoring agents, coloring matter or dyes and, if so desired, emulsifying and/or suspending agents as well, together with such diluents as water, ethanol, propylene glycol, glycerin and various like combinations thereof.

The following examples illustrate the invention but are not to be construed as limiting the same.

## EXAMPLE 1

BocPheHis-2,2-difluorocyclohexylSta N-methylamide (X = $\underset{\overset{\equiv}{OH}}{\overset{\backslash\ /}{CH}}$ and $R_1$ = $NHCH_3$)

1A. Ethyl 5-cyclohexyl-(S)-4-t-butoxycarbonylamino-(R)-3-hydroxy-2,2-difluoropentanoate and the 3-(S) epimer

To 0.798 g of zinc powder, activated by washing with aqueous hydrochloric acid, ethanol, ether and drying, in 4 ml of dry tetrahydrofuran was added with stirring at 25° C. 0.179 ml of titanium tetrachloride, and the resulting slurry was stirred for 15 minutes and cooled to 0° C. To this suspension was added by cannula over 3 minutes a solution of purified N-t-butoxycarbonyl-hexahydro-L-phenylalaninal (1.04 g) and 0.52 ml of ethyl bromodifluoroacetate in 4 ml of dry tetrahydrofuran. After stirring for 30 minutes at 25° C. a second portion of titanium tetrachloride (0.179 ml) was added. The mixture was allowed to stir for 15 minutes and was then poured, with vigorous stirring, into ether/ice/sodium bicarbonate solution. The ether extracts were combined, washed with a saturated sodium bicarbonate solution and a brine solution and then dried over sodium sulfate. The residue remaining after removal of the solvent *in vacuo* (1.4 g) was chromatographed on 130 g of silica using ethyl acetate-hexane (1:6, v:v) as the initial eluent. The eluent was changed to 1:5 of the same mixture to give 460 mg of the less polar 3-(R) isomer and 453 mg of the more polar 3-(S) isomer.

1B. 5-Cyclohexyl-(S)-4-t-butoxycarbonylamino-(R)-3-hydroxy-2,2-difluoropentanoic acid N-methylamide

A solution of 215 mg of ethyl 5-cyclohexyl-(S)-4-t-butoxycarbonylamino-(R)-3-hydroxy-2,2-difluoropentanoate in 2 ml of methanol was treated with an excess of methylamine gas at 25° C., and the reaction mixture, contained in a stoppered flask, was stirred for 2 hours. The reaction mixture was concentrated *in vacuo* and the residue was initially chromatographed on 10 g of silica using 500 ml of ethyl acetate-hexane (1:5, v:v) as the eluent followed by a second chromatography using the same eluent (1:1, v:v) to give 155 mg of the desired product.

1C. 5-Cyclohexyl-(S)-4-amino-(R)-3-hydroxy-2,2-difluoropentanoic acid N-methylamide hydrochloride

5-Cyclohexyl-(S)-4-t-butoxycarbonylamino-(R)-3-hydroxy-2,2-difluoropentanoic acid N-methylamide (139 mg) was dissolved in 2 ml of 4N hydrogen chloride-dioxane at 25° C. After one hour the mixture was concentrated under vacuum and the residue dried to give 119 mg of the product as a yellow powder.

1D. BocPheHis(imBoc)-2,2-difluorocyclohexylSta N-methylamide

To 115 mg of 5-cyclohexyl-(S)-4-amino-(R)-3-hydroxy-2,2-difluoropentanoic acid N-methylamide hydrochloride in 1 ml of methylene chloride at 0° C. was added 0.069 ml of triethylamine, 211 mg of BocPheHis(imBoc), 97 mg of 1-hydroxybenzotriazole and, after a few minutes of stirring, 87 mg of dicyclohexylcarbodiimide. The mixture was stirred at 0° C. for 5-6 hours and then allowed to warm to 25° C., where it was allowed to stir for an additional 12 hours. The mixture was filtered, the solids washed with methylene chloride, and the washings and filtrate combined and concentrated to dryness. The residue was redissolved in ethyl acetate

and the solution washed with a 1<u>N</u> sodium hydroxide solution (2 x 2 ml) and a brine solution, and dried over magnesium sulfate. The solvent was removed *in vacuo* and the residue chromatographed on 13 g of silica using 200 ml each of 0.5, 1.0, 2.0, 4.0 and 6.0% ethanol in methylene chloride. The appropriate fractions were combined and concentrated to dryness to give 209 mg of the product as a colorless foam.

1E. BocPheHis-2,2-difluorocyclohexylSta N-methylamide

To 200 mg of BocPheHis(imBoc)-2,2-difluorocyclohexylSta N-methylamide in 1.6 ml of glacial acetic acid was added 0.4 ml of water and the reaction mixture allowed to stir at 25° C. for 2.5 hours and then at 0° C. for 15 hours. The mixture was concentrated *in vacuo* and the residual material coevaporated with diethyl ether to give 180 mg of the product as a white powder.

$^1$H-NMR, DMSO-$d_6$, 250 mHz, partial, ppm from TMS: 1.32 (s, 9H, Boc); 2.69 (d, 3H, $CH_3N$); 3.38 and 4.17 (m, 2H ea); 3.97, 4.48, 8.09, and 8.82 (m, 1H ea); 6.87 and 7.58 (s, 1H ea, imidazolyl CH), also 7.58 (m, 1H), 7.08 (d, 1H); 7.15-7.35 (m, ca. 6H, aromatic).

## EXAMPLE 2

**BocPheHis-2,2-difluoro-epi-cyclohexylSta N-methylamide (X = $\underset{\blacktriangle OH}{\overset{\backslash\ /}{CH}}$ and $R_1 = NHCH_3$)**

**2A. 5-Cyclohexyl-(S)-4-t-butoxycarbonylamino-(S)-3-hydroxy-2,2-difluoropentanoic acid N-methylamide**

Starting with 122.5 mg of ethyl 5-cyclohexyl-(S)-4-t-butoxycarbonylamino-(S)-3-hydroxy-2,2-difluoropentanoate (Example 1A) and employing the procedure of Example 1B, with the exception the reaction mixture was stirred at 25° C. for 4 hours, the desired product was obtained in 97% yield as an off-white powder.

**2B. 5-Cyclohexyl-(S)-4-amino-(S)-3-hydroxy-2,2-difluoropentanoic acid N-methylamide hydrochloride**

Following the procedure of Example 1C and starting with 99 mg of 5-cyclohexyl-(S)-4-t-butoxycarbonylamino-(S)-3-hydroxy-2,2-difluoropentanoic acid N-methylamide the desired product was prepared in a quantitative yield as a yellow powder.

**2C. BocPheHis(imBoc)-2,2-difluoro-epi-cyclohexylSta N-methylamide**

The procedure of Example 1D was repeated exactly, starting with 82.6 mg of 5-cyclohexyl-(S)-4-amino-(S)-3-hydroxy-2,2-difluoropentanoic acid N-methylamide hydrochloride, to give the desired product in 62% yield as a colorless foam.

**2D. BocPheHis-2,2-difluoro-epi-cyclohexylSta N-methylamide**

Starting with 119.7 mg of BocPheHis(imBoc)-2,2-difluoro-epi-cyclohexylSta N-methylamide and following the exact procedure of Example 1E, the desired product was obtained in a quantitative yield as a colorless powder.

$^1H$-NMR, DMSO-$d_6$, 250 mHz, partial, ppm from TMS: 1.32 (s, 9H, Boc); 2.70 (d, 3H, $NCH_3$); 3.0 (m, 2-3H); 3.96-4.27 (overlapping m, 3H); 4.51 (q, 1H); 6.88 and 7.48 (s, 1H ea, imidazolyl CH); 7.1-7.35 (m, 6-8H, aromatic and NH); 7.84, 7.95, and 8.60 (m, 1H ea, NH).

## EXAMPLE 3

BocPheHis-2,2-difluorocyclohexylSta ethyl ester ($X = \underset{\equiv OH}{\overset{\diagdown\ \diagup}{CH}}$ and $R_1 = C_2H_5$)

3A. Ethyl 5-cyclohexyl-(S)-4-amino-(R)-3-hydroxy-2,2-difluoropentanoate hydrochloride

Ethyl 5-cyclohexyl-(S)-4-*t*-butoxycarbonylamino-(R)-3-hydroxy-2,2-difluoropentanoate (76 mg) was treated with 2 ml of 4*N* hydrogen chloride-dioxane at 25° C. for 30 minutes. The mixture was concentrated *in vacuo* to dryness giving 57 mg of the desired product as a colorless powder.

3B. BocPheHis(imBoc)-2,2-difluorocyclohexylSta ethyl ester

Following the procedure of Example 1D, 53 mg of ethyl 5-cyclohexyl-(S)-4-amino-(R)-3-hydroxy-2,2-difluoropentanoate in 0.5 ml of methylene chloride was neutralized with 0.03 ml of triethylamine and coupled to 89 mg of BocPheHis(imBoc) using 42 mg of 1-hydroxybenzotriazole and 36 mg of dicyclohexylcarbodiimide. The product, after purification by chromatography on 4 g of silica using 2% ethanol-methylene chloride as the eluent, amounted to 104 mg of a yellow foam.

3C. BocPheHis-2,2-difluorocyclohexylSta ethyl ester

BocPheHis(imBoc)-2,2-difluorocyclohexylSta ethyl ester (99 mg) was dissolved in 1.5 ml of acetic acid-water (4:1, v:v) and allowed to stir at 25° C. for 18 hours. The mixture was concentrated *in vacuo*, coevaporated with diethyl ether and dried to constant weight to give 87 mg of the product as a colorless foam.

In a similar manner, starting with ethyl 5-cyclohexyl-(S)-4-*t*-butoxycarbonylamino-(S)-3-hydroxy-2,2-difluoropentanoate and following the procedure of Example 3A-3C, BocPheHis-2,2-difluoro-epi-cyclohexylSta ethyl ester can be prepared.

$^1H$-NMR, DMSO-$d_6$, 300 mHz, partial, ppm from TMS: 1.27 (t, 3H, ethyl $CH_3$); 1.30 (s, 9H, Boc); 2.70, 3.90, and 4.44 (m, 1H ea); 2.90 and 4.16 (m, 2H ea); 4.29 (q, 2H, ethyl $CH_2$); 6.86 and 7.52 (s, 1H ea, imidazolyl CH); 7.05, 7.65, and 8.13 (d, 1H ea, NH); 7.15-7.35 (m, aromatic and NH, 6-7H).

## EXAMPLE 4

### BocPheHis-2,2-difluoro-3-oxocyclohexylSta N-methylamide ($R_2$ = $CH_3$)

To a solution of 46.0 mg of BocPheHis-2,2-difluorocyclohexylSta N-methylamide in 500 µl of dry methylene chloride and 150 µl of dimethylsulfoxide cooled to -50° C. was added 65 µl of trichloroacetic anhydride, and the reaction mixture allowed to stir for one hour at -50 to -55° C. Triethylamine (148 µl) was then added and the reaction mixture allowed to warm to room temperature. After 35 minutes the yellow solution was transferred to a separatory funnel to which was added 50 ml of methylene chloride. The organic phase was extracted with (2 x 2 ml) a saturated sodium bicarbonate solution, separated and dried over magnesium sulfate. Removal of the solvent <u>in</u> <u>vacuo</u> gave an orange oil which was chromatographed on a $C_{18}$ Sep-Pak® which was packed and loaded using 1:1 water-methanol (v:v). The sample was eluted with 5 ml of 1:1 water-methanol and then with 100% methanol, collecting 0.5 ml fractions. Fractions 11-16 were combined and the solvent removed to give 30.8 mg of the product as a yellow solid. $^1H$-NMR, (DMSO-$d_6$, 300 mHz, partial) ppm from TMS: 1.28 (s, 9H, Boc), 0.9 (m, 2H), 1.2 (m), 1.6 (m), 2.7 (bs, 3H, N-$CH_3$), 2.95 (m), 4.17 (m, 1H), 4.6 (m, 1H), 4.82 (m, 1H), 6.87 (m, 1H), 7.05 (m, 1H), 7.2 (m, aromatic), 7.58 (s, imidazole C-H), 8.2, 8.3, 8.5 and 9.15 (m, 1H each).

## PREPARATION A

### N-t-Butoxycarbonyl-L-phenylalanyl-L-N(im-t-butoxycarbonyl-histidine [BocPheHis(imBoc)]

A. Boc-L-phenylalanyl-L-histidine methyl ester

A slurry of 36.4 g L-histidine methyl ester dihydrochloride in dichloromethane (1 liter) was cooled to 5° C. and treated with 52 ml triethylamine. After 10 minutes 40 g Boc-L-phenylalanine was added followed by 1-hydroxybenzotriazole (30.6 g), then after another 5 minutes by dicyclohexylcarbodiimide (30.8 g), and the mixture was stirred at 0° C. for 4 hours and at 20° C. for 90 hours. The mixture was then filtered and the filtered solid was washed with dichloromethane, and the combined organic layers were concentrated and the residue was dissolved in 1 liter ethyl acetate. After 10 minutes of stirring the mixture was filtered and the filtrate was washed with 1N sodium hydroxide (3 x 150 ml), brine, dried over magnesium sulfate and concentrated giving 45.9 g of a colorless solid which was used without additional purification in Step B.

B. BocPheHis(imBoc)

Forty grams of the solid produced in Step A was dissolved in 600 ml methanol and 200 ml water was added. The chilled (0° C.) mixture was treated with 40 g anhydrous potassium carbonate, stirred at 15-20° C. for 2.5 hours, then at 28° C. for 1.5 hours, cooled to 10° C., and adjusted to pH 4.2 with 12N hydrochloric acid. The above solution was concentrated to about 250 ml and 70 ml water was added followed by 660 ml dioxane. The pH was brought at 0° C. to 13.5 and 29 ml di-t-butyldicarbonate was added. After 0.5 hour (during which time the temperature was raised to 20° C.) the pH had dropped to 9.5 and 10 ml di-t-butyldicarbonate was

added. After 1 hour the pH was 8.0 and the reaction was complete as measured by RP-HPLC. The mixture was concentrated to remove dioxane, 300 ml water was added, and the mixture was washed twice with ether, 500 ml ethyl acetate was added and the pH was brought at 10° C. to 1.2 with concentrated hydrochloric acid. The organic layer was separated and the aqueous layer was washed twice with ethyl acetate. The ethyl acetate layers were combined, washed with water, brine, dried over sodium sulfate, and concentrated to give after several coevaporations with ether and drying at 25° C. to constant weight a colorless foam, weight 44 g HPLC at 60/40 acetonitrile/pH 2.1 0.1M phosphate on Zorbax C-8 25 cm x 4.6 mm at 214 nm, 1.5 ml/min retention time 3.23 (94% of the total absorbence to 10 minutes).

0222523



CLAIMS FOR THE CONTRACTING STATES:
BE, CH, DE, FR, GB, IT, IL, LU, NL, SE

1. A compound of the formula

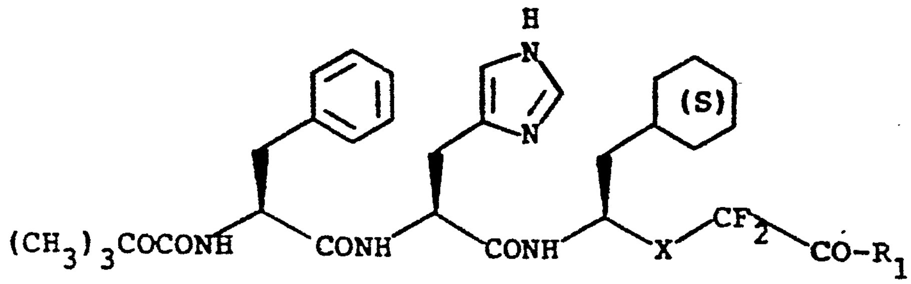

and a pharmaceutically acceptable acid addition salt thereof, wherein X is selected from the group consisting of

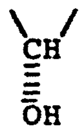 and 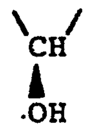

and $R_1$ is selected from the group consisting of alkoxy having one to three carbon atoms and alkylamino having one to three carbon atoms.

2. A compound of claim 1, wherein $R_1$ is alkylamino having one to three carbon atoms.

3. The compound of claim 2, wherein $R_1$ is methylamino and X is 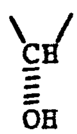 .

4. The compound of claim 2, wherein $R_1$ is methylamino and X is 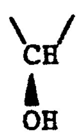 .

5. A compound of claim 1, wherein $R_1$ is alkoxy having one to three carbon atoms.

6. The compound of claim 5, wherein $R_1$ is ethoxy and X is 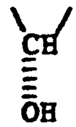 .

7. A compound of the formula

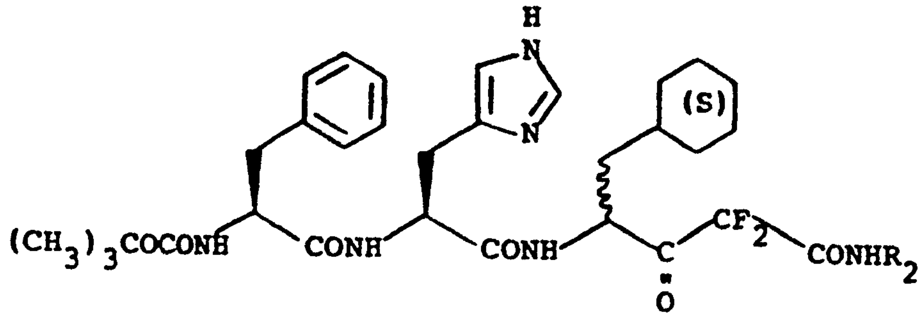

and a pharmaceutically acceptable acid addition salt thereof, wherein $R_2$ is alkyl having one to three carbon atoms.

8. The compound of claim 7, wherein $R_2$ is methyl.

CLAIMS FOR THE CONTRACTING STATES: AT ES GR

1. A process for preparing a compound of the formula

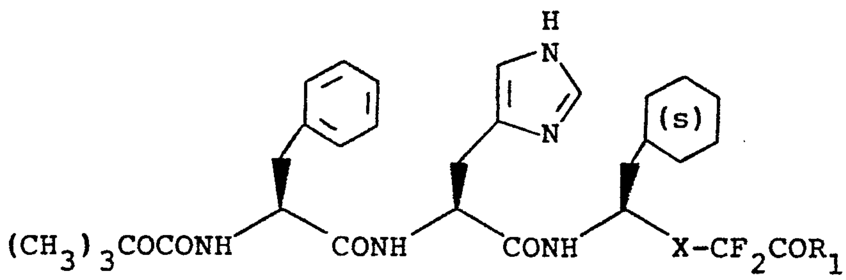

wherein $R_1$ is alkoxy of one to three carbon atoms or alkylamino of one to three carbon atoms and X is

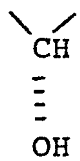

or

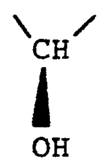

which comprises contacting a compound of the formula

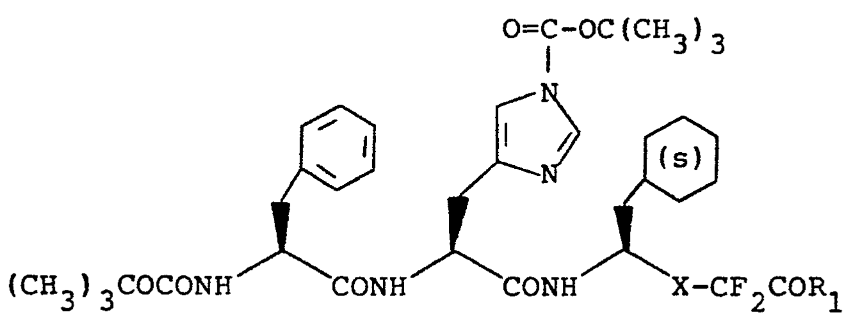

with glacial acetic acid - water at room temperature followed by isolation of the desired product.

2. The process of claim 1, wherein the glacial acetic acid - water ratio is 4:1 (v,v) and the product is isolated by evaporation of the reaction mixture.